# EUROPÄISCHE PATENTSCHRIFT

(11) **EP 0 870 062 B1**
(45) Veröffentlichungstag und Bekanntmachung des Hinweises auf die Patenterteilung: **13.02.2002**
(21) Anmeldenummer: 96946163.1
(22) Anmeldetag: 08.11.1996
(51) Int. Cl.: C12Q 1/68

(54) **VERFAHREN ZUR GENOMANALYSE UND MITTEL ZUR DURCHFÜHRUNG DES VERFAHRENS**
GENOMIC ANALYSIS PROCESS AND AGENT
PROCEDE ET AGENT D'ANALYSE GENOMIQUE

(30) Priorität: 09.11.1995 DE 19543065
(43) Veröffentlichungstag der Anmeldung: 14.10.1998
(73) Patentinhaber: GAG Bioscience Zentrum für Umweltforschung und Technologie, 28359 Bremen (DE)
(72) Erfinder: Olek, Alexander, 10781 Berlin (DE)
(74) Vertreter: Baumbach, Friedrich
(86) Internationale Anmeldenummer: DE9602169
(87) Internationale Veröffentlichungsnummer: WO9717461

(56) Entgegenhaltungen:
- WO-A-94/11530
- WO-A-94/25625
- WO-A-96/04676
- WO-A-96/32504
- CA-A- 2 013 430
- PROC. NATL. ACAD. SCI. USA, Bd. 89, November 1992, Seiten 10643-10647, XP002033264 TURNER B. ET AL.,: "Extreme clonal diversity and divergence in populations of a selfing hermaphroditic fish"
- NUCLEIC ACIDS RESEARCH, Bd. 23, Nr. 16, 25.August 1995, Seiten 3126-3131, XP002016562 TANG K ET AL: "MATRIX-ASSISTED LASER DESORPTION/IONIZATION MASS SPECTROMETRY OF IMMOBILIZED DUPLEX DNA PROBES"

## Beschreibung

Die Erfindung betrifft ein Verfahren zur Genomanalyse, insbesondere zur Analyse und Lokalisierung von erblich bedingten Eigenschaften im Genom. Es gibt eine große Zahl von Anwendungsgebieten für dieses Verfahren, insbesondere in der Medizin, der Landwirtschaft, Forensik und Grundlagenforschung.

### 1. Technischer Stand

### 1.1 Definition von Mikrosatelliten

Der Begriff des Mikrosatelliten wird hier in dem folgenden Sinne verwandt: ein Mikrosatellit ist ein Oligonucleotidrepeat, das zwischen den individuellen Chromosomen einer Species verschiedene Allele aufweist, also polymorph ist. Die verschiedenen Allele unterscheiden sich durch ihre Länge, das heißt durch ihr Molekulargewicht. Flankiert - und dadurch auch definiert - ist jeder Mikrosatellit durch zwei im Genom nur einmal vorkommende, nicht polymorphe DNA Sequenzen, die als Hybridisierungspartner für die Primer zur Polymerase Kettenreaktion benutzt werden.

### 1.2 Anwendungen von Mikrosatelliten Markern.

Mikrosatelliten Marker stellen ein hauptsächliches Werkzeug der modernen genetischen Analyse dar.

### 1.2.1

Zum einen werden diese Marker dazu benutzt, nach einer meiotischen Zellteilung zu ermitteln, welches der beiden Allele an einem mit dem betreffenden Marker assozierten genomischen Locus an einen Nachkommen weitergegeben wurde.

Dieses Prinzip wird bei der Kopplungsanalyse ausgenutzt: Werden Mikrosatelliten Marker Allele in einer Familie mit mehreren Betroffenen einer Erbkrankheit überdurchschnittlich häufig zusammen mit dem genetischen Defekt vererbt, sind Defekt Locus und Mikrosatelliten Marker physikalisch gekoppelt, das heißt sie liegen im Genom dicht beieinander.

Da die Lokalisierung eines Mikrosatelliten im Genom bekannt ist, läßt diese Information Rückschlüsse auf die Lage des (der) für den Defekt verantwortlichen Gene(s) zu. Zwei Methoden der Kopplungsanalyse werden zur Zeit praktisch realisiert.
- Die sogenannte Lod-score Methode basiert auf der Analyse mehrerer bis zu zahlreicher großer Familien mit mehreren Betroffenen,
- die "affected relative method" (ARM) auf dem Vergleich von sehr vielen Paaren von verwandten Probanden.

In beiden Fällen ist das Haplotypisieren zahlreicher Personen vonnöten. Unter Haplotypisieren wird verstanden, bei jeder zu analysierenden Person zu ermitteln, welche Allele eines jeden polymorphen Markers im Genom des Probanden vorhanden sind. Es werden heutzutage fast ausschließlich Mikrosatelliten Marker typisiert. Dies liegt daran, daß diese am zuverlässigsten und schnellsten analysiert (siehe Halbautomatisierung weiter unten) und auch am effizientesten identifiziert werden können. Der Stand der Technik ist daher, daß beim Menschen über 5000 dieser Marker zur Verfügung stehen und sich alle auf dem Markt befindlichen Systeme zur Kopplungsanalyse auf diese Marker stützen. Um mit einiger Sicherheit eine Kopplung eines Mikrosatelliten zu Genen zu etablieren, die für einen Defekt verantwortlich sind, müssen im Falle einer nach nicht mendelischem Muster vererbten Krankheit (diese Gruppe von Defekten und Prädispositionen ist das Hauptziel unserer Methode) mindestens 300 - 600 Paare betroffener Verwandter mit jeweils mindestens 400 Mikrosatelliten untersucht werden.

Ideal, und um nach Etablierung von Kopplungsgruppen eine möglichst genaue Vorstellung des/der gomischen Loci zu erhalten, ist das Genotypisieren von weitaus mehr Probanden mit wesentlich mehr Markern. In diesem zweiten Schritt werden Marker verwendet, die die im ersten Schritt durch Kopplung ermittelten Regionen mit wesentlich höherer Auflösung abdecken.
Der modernste und schnellste Ansatz zur Genotypisierung mit Mikrosatelliten ist das Verwenden von fluoreszenz-markierten Mikrosatelliten Primern und die Größenbestimmung der Mikrosatelliten-Allele auf einem halbautomatischen Sequenziergerät. Diese Methode läßt die Analyse von bis zu 24, im Durchschnitt aber nur 15 Mikrosatelliten auf einer Elektrophorese Gelbahn zu (mit bis zu 48 Bahnen pro Gel), was aber bedeutet, daß im besten Fall 1000-2000 solcher Gele gebraucht werden, um eine Kopplung mittels ARM zu etablieren. (Vignal,A et al., 1993; Methods in Molecular Genetics: Gene and Chromosome Analysis. Academic Press, San Diego. Seiten 211-221 und Davies,JL et al., 1994; Nature 371:130-136) Dies bedeutet, daß ein Sequenziergerät im Idealfall für mindestens ein Jahr ausgelastet ist. Die Kosten einer solchen Kopplungsanalyse einschließlich Personalkosten können bis zu mehrere Millionen DM betragen. In den letzten Jahren konzentriert sich das Interesse der beteiligten Wissenschaftler immer mehr auf häufige, polygene, multifaktorielle oder multigene Krankheiten (Lathrop,GM, 1993; Current opinion in Biotechnology 4:678-683). Je mehr Komponenten an der Entstehung einer Krankheit beteiligt sind, desto mehr Personen müssen zur Etablierung einer Kopplung genotypisiert werden; damit steigen Aufwand und Kosten exponentiell mit der Komplexität einer Krankheit. Dies bedeutet, daß eine Kopplungsanalyse für die sogenannten Volkskranheiten weitaus aufwendiger und teurer sein wird, als oben erwähnt.
Bei den Nutz- und Haustieren, aber auch Planzen ist die Kopplungsanalyse das vielversprechendste Instrument, um züchterisch wertvolle Eigenschaften im Genom zu lokalisieren und die zugehörigen Gene zu isolieren.

Das ermöglicht dem Züchter sowohl eine Konzentrierung positiver Eigenschaften als auch ein Minimieren von negativen Eigenschaften. Die bis heute gebräuchliche Züchtungsstrategie läßt die Beurteilung eines Zuchterfolges erst nach mehreren Generationen zu. Außerdem ist das Zuchtergebnis in den meisten Fällen nicht vorhersehbar.
Gerade bei den positiven Eigenschaften, wie zum Beispiel stabiler Resistenzen gegen Pathogene und Kälteresistenz handelt es sich in aller Regel um komplexe genetische Parameter, die den oben für humangenetische Fragestellungen beschriebenen Aufwand fordern.

### 1.2.2

Zweitens läßt sich ermitteln, ob ein bestimmtes Allel eines Mikrosatelliten überhaupt in zwei zu vergleichenden Genomen vorkommt und damit nach Analyse von genügend Markern eine Bestätigung oder ein Ausschluß einer Verwandtschaft möglich ist. Beim Menschen stellt sich diese Frage zum Beispiel bei der Vaterschaftsbestimmung oder in der Gerichtsmedizin zum Täterausschluß bzw. zur Täteridentifizierung. Außer der Typisierung der HLA Oberflächenantigene hat sich hier die Verwendung von hochpolymorphen Mikrosatelliten Markern in der Praxis durchgesetzt und die genetischen Fingerabdrücke (Multilokus) weitgehend ersetzt.
Auch bei Nutztieren ist der Herkunftsnachweis per Mikrosatellitenanalyse international zur Standardmethode erklärt worden.

In der Regel werden für diese Anwendungen etwa 10 Mikrosatelliten Marker analysiert. Der gesamte Nachweis, einschließlich der Auswertung erfordert etwa 2 Tage.

Die Kosten einer solchen Analyse betragen bei auch bei hohem Probenaufkommen zur Zeit mindestens 50 DM.

### 2. Nachteile der gegenwärtig gebräuchlichen Technologie

### 2.1 Kopplungsanalyse komplexer Krankheiten beim Menschen.

Für die Analyse von verbreiteten, multifaktoriellen Krankheiten wie z.B Arteriosklerose, Diabetes II, Alzheimer oder Schizophrenie ist die Analyse von bis zu mehreren tausend Paaren von verwandten Betroffenen erforderlich, da die Lod-score Methode in der Regel nicht angewandt werden kann. Die hauptsächlichen Gründe hierfür ist der Mangel an großen Familien mit genügend Betroffenen und das Fehlen eines Modells des Erbgangs der Krankheiten. Eine solche Analyse ist daher weltweit nur extrem wenigen Forschungsgruppen möglich. Der Kostenaufwand einer solchen Analyse ist sehr hoch, wie oben näher ausgeführt. Die Technik ist aber auch deswegen teuer, weil die Herstellungskosten fluoreszenzmarkierter Primer extrem hoch sind. Man ist bei der Synthese dieser Primer von einigen wenigen Firmen abhängig.

Ein prinzipieller Nachteil der elektrophoretischen Auftrennung von fluoreszenzmarkierten DNA Fragmenten besteht darin, daß keine wirklich automatische Probenbearbeitung möglich ist. Noch schwieriger ist die Automatisierung der Auswertung. Die von der Herstellern der Sequenziergeräte konzipierte Soft-Ware ist zwangsläufig unzulänglich. Im Grunde bemüht man sich mit den kompliziertesten EDV-Mitteln um die Automatisierung einer Methode, die nicht zu automatisieren ist.

Ein entscheidender Nachteil der gebräuchlichen, elektrophoretischen Methoden besteht darin, daß keine absolute Größenbestimmung von Fragmenten möglich ist.
Dies bedeutet, daß aufwendige und letztlich doch nicht immer verläßliche Kalibrierungsprozeduren benötigt werden. Einen Teil dieser Probleme versucht man durch den Einsatz klonierter Allele als interne Standards auszuräumen. Das mag auch in Grenzen gelingen.

Noch viel schwieriger ist allerdings der Umstand zu bewältigen, daß die Mobilität der den Allelen entsprechenden DNA-Fragmente von diversen Parametern der Probenaufarbeitung abhängig ist, so z.B. der Markierung und einzelnen Reaktionsbedingungen in der PCR. Damit muß man also die entscheidende Meßgröße der Mikrosatelliten-Analyse, nämlich die Fragmentlänge durch eine Reihe von Hilfsgrößen stützen. Nur einem sicher identifizierten Allel kann man aber in der Identitäts- oder Herkunftsanalyse eine bestimmte Frequenz zuordnen. Von letzterer hängt aber fast ausschließlich die Aussagesicherheit einer Bestimmung ab.

### 2.2. Kopplungsanalyse bei Nutztieren und Pflanzen

Die Lokalisierung von Genen, die züchterisch wertvolle Eigenschaften steuern, verbietet sich bei Nutz- und Haustieren und Pflanzen in der Regel auf Grund des extrem hohen Preises der Studie. Die weitaus meisten züchterisch interessanten Eigenschaften haben einen polygenen Hintergrund, so daß eine sehr aufwendige und sehr teure Kopplungsanalyse erforderlich wäre.

### 2.3. Herkunftsbestimmung bei Nutztieren.

Auch hier ist der bestimmende Faktor der Preis einer Analyse. Herkunftsbestimmung bei Nutztieren ist sinnvoll, wenn jedes Tier individuell genotypisiert wird oder zumindest große Mengen von Stichproben analysiert werden können. Bei den meisten Nutztierspezies macht der Preis einer konventionellen Mikrosatelliten Analyse einen beträchtlichen Teil des Preises eines Tieres aus.

Weder Züchter noch Verbraucher sind bereit, eine erhebliche Verteuerung von Nutztieren hinzunehmen, was die Mikrosatelliten Analyse von diesen Tieren marktwirtschaftlich sinnlos macht.

### 3. Aufgabe der Erfindung

Der Erfindung liegt die Aufgabe zugrunde, die Analyse von Genomen, insbesondere die Lokalisierung von genetisch bedingten Eigenschaften im Genom, zu rationalisieren und damit auch eine Anwendung in Fällen zu ermöglichen, bei denen die bisherigen Methoden aus Kostengründen nicht in Betracht kommen.

### 4. Wesen der Erfindung

Das erfindungsgemäße Verfahren ist durch den Hauptanspruch gekennzeichnet, die Unteransprüche sind Vorzugsvarianten. Es sieht das Analysieren von Mikrosatelliten mittels Massenspektrometrie vor. Dazu sollen die jeden Mikrosatelliten flankierenden einzigartigen Sequenzen als einzelsträngige DNA auf einer festen Matrix fixiert werden. Die Matrix ist so unterteilt, das jede einen Mikrosatelliten definierende einzelsträngige DNA an einem bestimmten Punkt dieser Matrix befestigt wird. Wird nun eine Mischung von amplifizierten Mikrosatelliten im einzelsträngigem Zustand auf diese Matrix gegeben, hybridisieren die vorhandenen Allele jedes Mikrosatelliten nur genau an dem Punkt der Matrix, an dem die diesen Mikrosatelliten definierende Primer-Sequenz angebracht wurde.

Das Kennzeichen dieser Variante der erfindungsgemäßen Methode ist, daß im Prinzip nur ein einziger PCR Ansatz benötigt wird, um (im Idealfall) alle Mikrosatelliten zu amplifizieren, die als Gesamtheit zur Hybridisierung mit der Matrix in Kontakt gebracht werden (Multiplex PCR mit allen verwendeten Mikrosatelliten Primern). Selbst wenn dieser Idealfall nicht gegeben ist und nicht alle Mikrosatelliten in der selben Reaktion amplifiziert werden können, sind sehr viel weniger Reaktionen nötig um alle Mikrosatelliten, die z.B für eine Kopplungsanalyse benötigt werden, zu amplifizieren.
Fakt ist, daß die Limitierung von heutigen Methoden (siehe Stand der Technik, Multiplex PCR mit fluoreszenz-markierten Primern) hauptsächlich auf darauf beruht, daß nicht mehr als maximal 24 Fragmente nichtüberlappend auf einer Gelbahn aufgelöst werden können. Auf die Hybridisierung der Mikrosatelliten Amplifikate folgt erfindungsgemäß das schrittweise Lösen der DNA von den einzelnen Punkten der Matrix und das Auftragen der DNA auf die Sonde des Massenspektrometers. Diese Prozedur kann automatisch mit sehr großer Geschwindigkeit erfolgen (siehe z.B. Köster, Human Genome Mapping Symposium, Cold Spring Harbour Laboratory, 1995). Moderne Ansätze zur DNA Sequenzierung mittels Hybridisierung und massenspektro-metrischer Analyse lassen das Auftragen auf ein Massenspektrometer von Hunderten von Fragmenten in der Größenordnung von Millisekunden zu.
Die Matrix kann eine feste Phase darstellen, an der die Oligonukleotide per kovalenter Bindung, Hybridisierung oder andere Arten physikalischer oder chemischer Bindungen fixiert werden. Sie kann auch dargestellt werden durch Mikrotiterplatten oder andere Gefäße, in deren einzelne Reaktionsgefäße die Locus-spezifischen Oligonukleotide fixiert werden.
Eine weitere Ausführungsform der Erfindung besteht darin, die Mikrosatelliten einzeln, in sehr kleinem Reaktionvolumen zu amplifizieren.

Neuartige Nanopipettiersysteme lassen das computergesteurte Ansetzen von Reaktionen im Maßstab von weniger als 1µl zu. Hunderte oder Tausende von PCR Reaktionen können so simultan auf miniaturisierten Microtiterplatten oder in ähnlich miniaturisierten Reaktionsgefäßen erfolgen. Der Unterschied dieser Variante im Gegensatz zu der oben genannten ist, daß Multiplex PCR (das Amplifizieren von vielen, bis Hunderten von Mikrosatelliten) zwar durchgeführt werden kann (siehe Stand der Technik) aber in der Praxis zu Problemen bei der Reproduzierbarkeit der Ergebnisse führen könnte, wenn extrem komplexe Reaktionen (mehrere hundert Primerpaare) durchgeführt werden.
Daher soll auch die folgende Analysestrategie von Mikrosatelliten im Massenspektrometer geschützt werden: Computergesteuerte Nanoliterpipetten bedienen PCR-Reaktionsgefäße einer Dimension < 1µl. Diese Pipetten bringen die einzelnen Marker-Loci-Amplifikate auf eine Matrix auf, die die amplifizierte DNA in einer Art und Weise absorbiert, daß diskrete Punkte der Matrix mit einzelnen Amplifikaten versehen werden können, ohne daß sich Proben von benachbarten Punkten der Matrix vermischen. Verschiedene handelsübliche Matrices verfügen über diese Eigenschaft (z.B Nitrozellulose oder Nylon). Moderne Pipettiersysteme sind in der Lage, einige tausend Proben im Nanoliterbereich pro cm² aufzutragen. Da die Mikrosatelliten einzeln amplifiziert werden, kann auf jeden definierten Punkt der Matrix ein definiertes Mikrosatelliten Amplifikat aufgetragen werden. Von dieser Matrix werden die Amplifikate nacheinander verdampft und massenspektrometrisch verarbeitet.
Beide Ausführungsvarianten leisten daher ähnliches: auf engstem Raum werden an definierten Stellen Mikrosatelliten Amplifikate fixiert, die daraufhin im Massenspektrometer auf ihr Molekulargewicht analysiert werden.

Die Automatisierung wird dabei angestrebt, das Analysieren von Mikrosatelliten im Massenspektrometer soll aber im allgemeinen geschützt werden. Zur Erfindung gehört auch das massenspektrometrische Analysieren direkt aus Mikrosatelliten Amplifikations-reaktionen. Auch ohne das vorherige Fixieren der Mikrosatelliten auf einer Matrix auf eine der beiden genannten Verfahrensvarianten wäre das manuelle Analysieren von Mikrosatelliten Markern im Massenspektrometer ein Vorteil gegenüber gebräuchlichen Verfahren.

Im Prinzip taugt jedes der verfügbaren unterschiedlichen massenspektrometrischen Verfahren für die hier beschriebene Analysenmethode, wenn damit hochmolekulare Biomoleküle untersucht werden können.
Das massenspektrometrische Verfahren beruht auf dem Auftragen des Mikrosatelliten Marker Amplifikats auf eine Sonde (entweder die oben genannten Matrices oder direkt aus der Lösung auf die Sonde des Spektrometers). Als Sonde ist jede Oberfläche definiert, die in den Verdampfungsraum des Massenspektrometers eingeführt werden kann und von der aus eine zu analysierende Probe in die Gasphase gebracht werden kann. Die aufgetragene DNA wird daraufhin in eine Dampfphase gebracht und durch eine elektrisches oder magnetisches Feld beschleunigt. Die Bahn der beschleunigten DNA führt an einem elektrischen oder magnetischen Feld vorbei, das die DNA von der geraden Flugbahn ablenkt. Der Grad dieser Ablenkung ist von der Ladung (bei DNA pro atomare Gewichtseinheit konstant) und vom Molekulargewicht der DNA abhängig. Der Grad der Ablenkung, und daher das absolute Molekulargewicht kann durch die Position des Aufschlags des DNA Moleküls auf einen Detektor ermittelt werden.

Da die Auswertung einer massenspektrometrischen Analyse in jedem Fall direkt digitalisiert im angeschlossenen Computer erfolgt, kann das Molkulargewicht aller Mikrosatelliten Allele direkt an ein genetisches Analyseprogramm weitergegeben werden.

### 5. Vorteile des erfindungsgemäßen Verfahrens

Das Verfahren ist billiger als jedes andere Verfahren zur Mikrosatelliten Analyse. Es ist genauer; es gestattet eine objektive Massenbestimmung mit extrem hoher Auflösung.

Für die Analyse eines Locus werden Sekunden anstatt von Stunden benötigt. Es werden keine speziell markierten DNA Sonden oder Primer benötigt, die einen erheblichen Kostenaufwand bedeuten. Zwar sind Massenspektrometer ähnlich kostspielig wie automatische Sequenziergeräte, die Analyse ist aber so schnell, das auch technologisch nicht ausreichend ausgestattete Institutionen eine Kopplungsanalyse als Serviceleistung in Auftrag geben können.
Diese Vorteile haben zur Folge, daß eine Kopplungsanalyse bei der Genomanalyse des Menschen erleichtert, beschleunigt und erheblich billiger angewendet werden kann. Dies ist eine Voraussetzung für die Analyse von sehr komplexen genetischen Defekten und Prädispositionen. Außerdem wird das Verfahren des Studiums von Tiermodellen von menschlichen Krankheiten (bei denen eine unbegrenzte Anzahl von Individuen zur Analyse zur Verfügung steht, da planmäßiges Kreuzen möglich ist) so erheblich erleichtert, daß dieser Ansatz erheblich an Praktikabilität gewinnen wird.

Die routinemäßige Lokalisierung von züchterisch wertvollen Merkmalen bei Nutztieren wird durch das erfindungsgemäße Verfahren erst möglich gemacht. Der Preis und der Zeitaufwand einer solchen Analyse übersteigen zur Zeit aus analytischen Günden (siehe oben) den Nutzen der Analyse. Gleiches gilt auch für das Kartieren von z.B. Resistenzen und anderen Eigenschaften bei Nutzpflanzen. Erst wenn eine effiziente Methode zur Mikrosatelliten Analyse auf dem Markt ist, werden systematische Genomprojekte die Voraussetzung schaffen, routinemäBig pflanzliche Eigenschaften zu kartieren.
Die Herkunfts- und Identitätsanalyse beim Menschen wird technisch und juristisch sicherer werden. Eine absolute Größenbestinmung von Mikrosatelliten Markern wird möglich gemacht und damit ein hauptsächliches Problem dieser Methode in diesem Bereich ausgeräumt.

Die Herkunfts- und Identitätsanalyse bei den meisten Nutztieren und Pflanzen wird erst durch die Erfindung ermöglicht, da zum ersten Mal der Preis einer solchen Analyse keinen signifikanten Anteil am Marktwert des Tieres/der Pflanze hat.
Ein erheblicher Vorteil ist, daß alle Informationen direkt aus dem Massenspektrometer mittels eines dafür konzipierten Interfaces an gebräuchliche Analyse-Software übermittelt werden,um zum Beispiel eine Kopplung zu etablieren. Dies spart die Kosten und Unsicherheiten eines, heute unabkömmlichen manuellen Schrittes.

## Patentansprüche

1. Verfahren zur Mikrosatellitennanlyse, **gekennzeichnet durch**
- die Fixierung von Mikrosatelliten Amplifikaten aus genomischen DNA-Proben, welche vor oder nach der Amplifikation in individuelle Mikrosatelliten-Marker getrennt werden, auf definierten Positionen einer Matrix,
- Verdampfen der einzelnen Positionen in einem Massenspektrometer und
- massenspektrometrische Bestimmung des Molekulargewichts.

2. Verfahren gemäß Anspruch 1, **dadurch gekennzeichnet,**
**daß** Mikrosatelliten Amplifikate aus einer Multiplex-PCR analysiert werden.

3. Verfahren gemäß Anspruch 2, **dadurch gekennzeichnet,**
**daß** die Fixierung der Amplifikate durch Hybridisierung mit an definierten und distinkten Punkten kovalent oder nicht-kovalent fixierten, die individuellen Mikrosatelliten definierenden Oligonucleotiden erfolgt.

4. Verfahren gemäß Anspruch 2, **dadurch gekennzeichnet,**
**daß** die Fixierung der Amplifikate durch Hybridisierung in an definierten und distinkten Punkten angeordneten Reaktionsgefäßen mit an definierten Einheiten kovalent oder nicht-kovalent fixierten, die individuellen Mikrosatelliten definierenden Oligonucleotiden erfolgt.

5. Verfahren gemäß Anspruch 1, **gekennzeichnet durch**
- Fixierung der Mikrosatelliten Amplifikate aus PCR-Reaktionen einzelner Mikrosatelliten Marker an eine Matrix mittels automatisierbarem Auftragen an definierte und distinkte Punkte dieser Matrix,
- nachfolgende Einführung dieser Matrix als Sonde in den Verdampfungsraum eines Massenspektrometers und
- automatisierte massenspektrometrische Molekulargewichtsbestimmung.

## Claims

1. Method for microsatellite analysis, wherein there exist
- the fixation of microsatellite amplificates from genomic DNA samples separated into individual microsatellite markers before or after amplification to defined positions of a matrix,
- evaporation of the individual positions in a mass spectrometer and
- mass-spectrometry determination of the molecular weight.

2. Method according to Claim 1, wherein
microsatellite amplificates from a multiplex PCR are analysed.

3. Method according to Claim 2, wherein
the fixation of the amplificates is done by hybridisation with oligo-nucleotides fixed covalently or non-covalently at defined and distinct points and defining the individual microsatellites.

4. Method according to Claim 2, wherein
the fixation of the amplificates is done by hybridisation in reaction vessels arranged at defined and distinct points with oligo-nucleotides fixed covalently or non-covalently at defined units and defining the individual microsatellites.

5. Method according to Claim 1, wherein there exist
- fixation of the microsatellite amplificates from PCR reactions of individual microsatellite markers to a matrix by means of automation-capable application to defined and distinct points of said matrix,
- subsequent introduction of said matrix into the evaporation area of a mass spectrometer as a probe and
- automated mass-spectrometry determination of molecular weight.

## Revendications

1. Procédé d'analyse de microsatellites, se caractérisant par
- la fixation d'amplificateurs microsatellites issus d'échantillons d'ADN génomiques qui sont séparés avant ou après l'amplification en marqueurs microsatellites individuels sur des positions définies d'une matrice,
- l'évaporation des différentes positions dans un spectromètre de masse et
- la détermination au niveau de la spectrométrie de masse du poids moléculaire.

2. Procédé conformément à la revendication 1, se caractérisant par le fait
que les amplificateurs microsatellites sont analysés à partir d'une PCR Multiplex.

3. Procédé conformément à la revendication 2, se caractérisant par le fait
que la fixation des amplificateurs se réalise par hybridation avec des oligonucléotides définissant les microsatellites individuels et fixés en covalence ou non-covalence à des points définis et distincts.

4. Procédé conformément à la revendication 2, se caractérisant par le fait
que la fixation des amplificateurs se réalise par hybridation dans des récipients réactionnels affectés à des points définis et distincts, avec des oligonucléotides définissant les microsatellites individuels et fixés en covalence ou non-covalence à des unités définies.

5. Procédé conformément à la revendication 1, se caractérisant par
- la fixation des amplificateurs microsatellites issus des réactions PCR des différents marqueurs microsatellites sur une matrice au moyen de l'application qui peut être automatisée sur des points définis et distincts de cette matrice,
- l'introduction subséquente de cette matrice en tant que sonde dans l'espace d'évaporation d'un spectromètre de masse et
- la détermination automatisée au niveau de la spectrométrie de masse du poids moléculaire.
